# EUROPÄISCHE PATENTSCHRIFT

(11) **EP 0 871 403 B1**
(45) Veröffentlichungstag und Bekanntmachung des Hinweises auf die Patenterteilung: **16.01.2002**
(21) Anmeldenummer: 96942261.7
(22) Anmeldetag: 22.11.1996
(51) Int. Cl.: A61B 17/00

(54) **VORRICHTUNG ZUR DURCHFÜHRUNG VON MANIPULATIONEN IM MENSCHLICHEN KÖRPER UND INSBESONDERE IM UTERUS**
DEVICE FOR CARRYING OUT MANIPULATIONS IN THE HUMAN BODY, IN PARTICULAR IN THE UTERUS
DISPOSITIF POUR EFFECTUER DES MANIPULATIONS A L'INTERIEUR DU CORPS HUMAIN, NOTAMMENT DE L'UTERUS

(30) Priorität: 22.11.1995 DE 19543576
(43) Veröffentlichungstag der Anmeldung: 21.10.1998
(73) Patentinhaber: Karl Storz GmbH & Co. KG, 78532 Tuttlingen (DE)
(72) Erfinder: SCHIFF, Norman, D-88709 Meersburg (DE); DITTRICH, Horst, D-78194 Immendingen (DE)
(74) Vertreter: Münich, Wilhelm, Dr.
(86) Internationale Anmeldenummer: DE9602251
(87) Internationale Veröffentlichungsnummer: WO9718757

(56) Entgegenhaltungen:
- WO-A-94/20034
- US-A- 4 633 869

## Beschreibung

### Technisches Gebiet

Die Erfindung bezieht sich auf eine Vorrichtung zur Durchführung von Manipulationen im menschlichen Körper und insbesondere im Uterus gemäß dem Oberbegriff des Patentanspruchs 1.

### Stand der Technik

Eine Vorrichtung zur Durchführung von Manipulationen im menschlichen Körper und insbesondere im Uterus gemäß dem Oberbegriff des Patentanspruchs 1 ist aus der US-PS 4,633,869 bekannt. Das Gleitelement dieser Vorrichtung, das auf dem Schaft in dessen Längsrichtung verschiebbar geführt ist, hat eine Erweiterung am distalen Ende, die aus flexiblem Material besteht. Das so ausgebildete Gleitelement dient in der Regel dazu, an eine Körper-Außenfläche angelegt zu werden, um die Zange zu fixieren. Dieser Teil des Instrument ist nicht in den menschlichen Körper einsetzbar.

Aus der WO 94/20034 ist eine medizinische Zange bekannt, wie sie beispielsweise in Verbindung mit glockenförmigen Gleitelementen eingesetzt werden kann. Zu der gleichen Patentfamilie gehört die EP 0688187, die dieser Veröffentlichung entspricht, die aber erst nach dem Anmeldetag der vorliegenden Anmeldung publiziert wurde.

Darüber hinaus ist es bekannt, am distalen Ende ein Hakenelement vorzusehen, dessen Haken über eine Stange, die in einem Schaft geführt ist, mit am proximalen Ende der Vorrichtung vorgesehenen Griffstücken verbunden ist. Durch Ausspreizen und Einziehen der Haken können Manipulationen im menschlichen Körper, und insbesondere im Uterus vorgenommen werden.

Sowohl das aus der WO 94/20034 bekannte Instrument als auch die bekannten Instrumente mit Haken haben in manchen Anwendungsfällen den Nachteil, dass es schwierig ist, die gewünschte Manipulation durchzuführen, da die Gewebeteile, die mit dem Zangenmaul oder mit dem oder den Haken manipuliert werden sollen, zur Seite ausweichen können.

### Darstellung der Erfindung

Der Erfindung liegt die Aufgabe zugrunde, eine gattungsgemäße Vorrichtung zur Durchführung von Manipulationen im menschlichen Körper und insbesondere im Uterus derart weiterzubilden, dass sie in den Körper einsetzbar ist und auch in den Fällen, in denen die zu manipulierenden Gewebeteile seitwärts ausweichen, die gewünschte Manipulation ausgeführt werden kann.

Eine erfindungsgemäße Lösung dieser Aufgabe ist im Patentanspruch 1 angegeben.
Das erfindungsgemäße Instrument kann danach wie folgt eingesetzt werden:
Durch Verschieben des auf dem Schaft vorgesehenen Gleitelements mit der angenähert glockenförmig ausgebildeten Erweiterung, und durch Betätigen der distal vorgesehenen Elemente, also beispielsweise des oder der Haken, können die jeweiligen Gewebeteile manipuliert werden. Insbesondere ist es möglich, mit dem Gleitelement das Gewebe in Zusammenwirken mit dem oder den Haken "festzuhalten" .

Gemäß Anspruch 2 weist das Gleitelement eine Beleuchtungseinrichtung auf, deren Lichtaustritt am Umfangsrand der glockenförmigen Erweiterung vorgesehen ist. Hierdurch ist es möglich, den zu manipulierenden Gewebeabschnittgegebenenfalls zusätzlich zu einer Endoskop-Beleuchtungzu beleuchten. Vor allem aber ist es möglich, laparoskopisch die Position des Glockenrandes zu bestimmen. Durch die Beleuchtung ist dabei genau die Position des Gleitelements für den Operateur sichtbar.

Die im Anspruch 3 angegebene Weiterbildung, gemäß der die Erweiterung in etwa die Form einer Halbkugel hat, hat den Vorteil, dass sich die Erweiterung bei der Entnahme oder beim Zurückziehen nicht im Gewebe oder der Körperöffnung verhaken kann.

Erfindungsgemäß ist gemäß Anspruch 4 weiter eine ergonomisch besonders vorteilhafte Ausführungsform angegeben, bei der eine Antriebseinheit für das Gleitelement vorgesehen ist, die ein Handrad aufweist, das eine in einer Längsnut geführte Kugel über eine gewindeförmige Nut bewegt. Damit muss die Bedienungsperson zur Längsverschiebung des Gleitelements nur eine Drehbewegung der Hand ausführen.

Das Hakenelement kann in an sich bekannter Weise wenigstens drei, bevorzugt vier Haken aufweisen, die bei geöffneten Griffstöcken eingeklappt sind, und die durch das Schließen der Griffstücke durch die als Zugstange wirkende Stange in Richtung auf das distale Ende ausgeklappt werden.
(Anspruch 5)

Durch die in den Ansprüchen 6 und 7 angegebenen Weiterbildungen werden die Reinigung und die Sterlisierung der erfindungsgemäßen Vorrichtung erleichtert. Das Hakenelement, der Schaft und der Griff können dabei insbesondere in einer Weise miteinander verbunden sein, wie dies in der EP-A-0 688 187 beschrieben ist.

### Kurze Beschreibung der Zeichnung

Die Erfindung wird nachstehend anhand der Zeichnung näher beschrieben, in der zeigen:
- Fig. 1: eine Einzelteildarstellung eines erfindungsgemäß ausgebildeten Uterusmanipulators,
- Fig. 2: den zusammengebauten Uterusmanipulator mit geschlossenem Hakenelement,
- Fig. 3a: den in Figur 2 dargestellten Uterusmanipulator mit geöffnetem Hakenelement, und
- Fig. 3b: eine perspektivische Darstellung des Hakenelements mit vier Haken.

### Beschreibung eines Ausführungsbeispiels

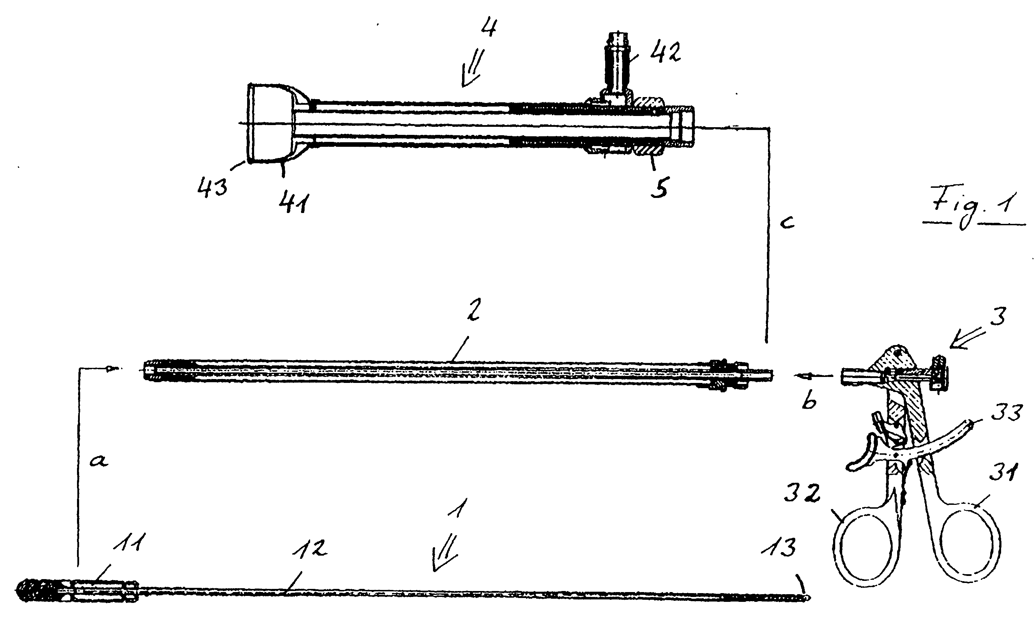

Fig. 1 zeigt die wesentlichen Bestandteile einer erfindungsgemäßen Vorrichtung zur Durchführung von Manipulationen im menschlichen Körper, die sich insbesondere als Uterusmanipulator eignet. Die Vorrichtung weist eine Hakeneinheit 1 auf, die an ihrem distalen Ende ein Hakenelement 11 trägt, das im folgenden noch näher erläutert werden wird. Das Hakenelement 11 ist über eine (Betätigungs)-Stange 12 mit dem proximalen Ende des Uterusmanipulators verbunden. Am proximalen Ende der Stange 12 ist eine Kugel 13 vorgesehen, deren Funktion nachstehend noch erläutert werden wird. Die Hakeneinheit 1 wird in einen Schaft 2 der Vorrichtung eingesetzt. Dies ist durch den Pfeil a versinnbildlicht. Die Verbindung zwischen Hakeneinheit 1 und Schaft 2 erfolgt über eine Bajonettverbindung. Als Verdrehsicherung sind am proximalen Ende gefederte Halbschalen vorgehen, wie dies beispielsweise in der EP-A-0 688 187 beschrieben ist.

Mit dem proximalen Ende des Schafts 2 wird ein Griffstück 3 mit zwei Griffen 31 und 32 und einer Raste 33 - beispielsweise durch eine Schraubverbindung - aber auch über jede andere geeignete Verbindung - verbunden (siehe Pfeil b). Durch die Raste 33 sind die Griffe 31 und 32 in einer bestimmten Stellung festlegbar. Die Kugel 13 wird durch eine rastbare Kugelverbindung mit dem beweglichen Griff 32 des Griffstücks 3 verbunden.

Soweit wie vorstehend beschrieben ist das Instrument im wesentlichen durch die sog. Take-apart-Zangen der Karl Storz GmbH & Co. oder die genannte EP-A-0 688 187 bekannt, so daß auf eine nähere Erläuterung verzichtet werden kann und statt dessen auf den genannten Stand der Technik Bezug genommen wird.

Erfindungsgemäß ist ein Gleitelement 4 vorgesehen, das an seinem distalen Ende eine glockenförmige Erweiterung 41 aufweist. Im proximalen Bereich des Gleitelements 4 ist ein Stutzen 42 für den Anschluß einer Beleuchtungseinrichtung vorgesehen. Das von der Beleuchtungseinrichtung emittierte Licht wird durch (in der Endoskopie bekannten) Lichtleiter zum distalen Ende geführt. Der Lichtaustritt ist im Umfangsrand 43 der Erweiterung 41 vorgesehen.

Das Gleitelement 4 wird vor der Verbindung der Hakeneinheit 1 mit dem Schaft 2 auf den Schaft 2 aufgesetzt (Pfeil c). Anschließend wird das Griffstück 3 mit dem Außenschaft 2 und dem proximalen Ende der Stange 12 verbunden.

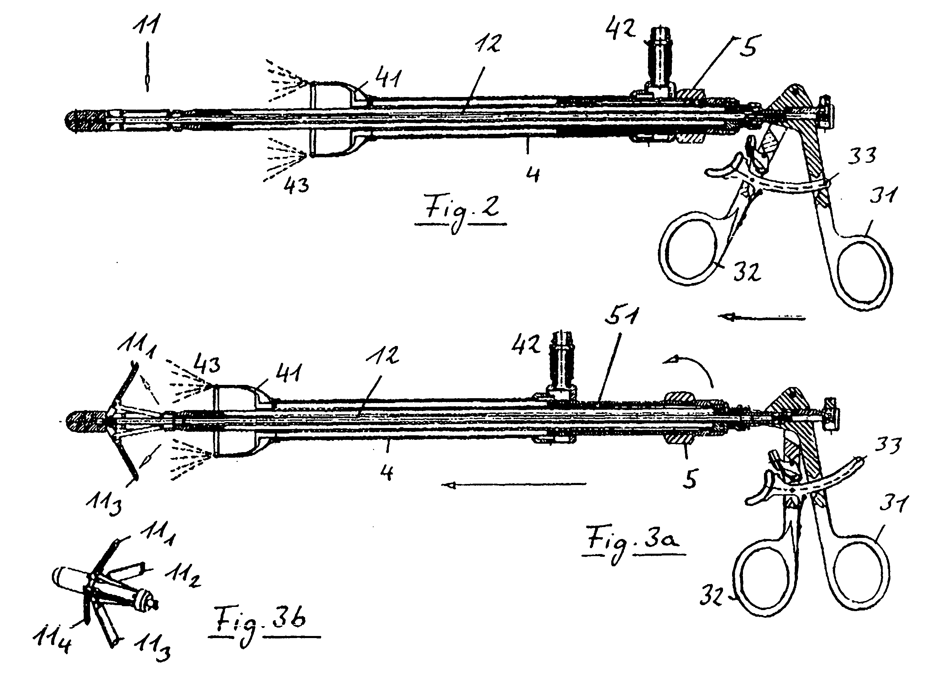

In den Figuren 2 und 3 ist der erfindungsgemäß ausgebildete Uterusmanipulator zusammengebaut dargestellt:

Das Hakenelement 11, das - wie Figur 3b zeigt - vier Haken 11₁ bis 11₄ aufweist, ist so ausgebildet, daß die Haken bei geöffneten Griffen 31 und 32 eingeklappt sind. Durch Schließen bzw. Zusammendrücken der Griffstücke werden die Haken durch die als Zugstange wirkende Stange 12 in Richtung auf das distale Ende ausgeklappt. Zur Durchführung der gewünschten Manipulationen wird das Gleitelement 4 durch Drehen eines Handrades 5 in Richtung der Längsachse des Schaftes 2 verschoben. Hierzu ist eine Kugel 51 vorgesehen, die in einer Längsnut geführt ist und die über eine gewindeförmige Nut in Richtung der Längsachse des Instruments bzw. des Manipulators verschoben wird.

Durch Vor- und Zurückziehen des Gleitelements 4 kann die mit den Haken 11₁ bis 11₄ durchgeführte Manipulation unterstützt werden. Zusätzlich tritt Beleuchtungslicht aus dem Umfangsrand der glockenförmigen Erweiterung 41 aus, um beispielsweise laparoskopisch die Position des Glockenrandes des vaginal eingeführten Instruments bestimmen zu können.

Vorstehend ist die Erfindung ohne Beschränkung des allgemeinen Erfindungsgedankens beschrieben worden, innerhalb dessen selbstverständlich die verschiedensten Abwandlungen möglich sind: So kann das Grundinstrument anders als die beschriebene, bevorzugte Ausführungsform aufgebaut sein. Auch kann der Vorschub des Gleitelements anders als beschrieben erfolgen.

## Patentansprüche

1. Vorrichtung zur Durchführung von Manipulationen im menschlichen Körper und insbesondere im Uterus, mit einem Hakenelement (11), das wenigstens einen Haken aufweist, der über eine Stange (12), die in einem Schaft (2) geführt ist, mit einem am proximalen Ende vorgesehenen Griffstück (3) verbunden ist, so dass er aus- und einschwenkbar ist, und einem Gleitelement (41), das auf dem Schaft (2) in dessen Längsrichtung verschiebbar geführt ist, und das an seinem distalen Ende eine angenähert glockenförmige Erweiterung (41) aufweist,
**dadurch gekennzeichnet, dass** die glockenförmige Erweiterung am distalen Ende des Gleitelements (41) derart ausgebildet ist, dass sie in den menschlichen Körper einfuhrbar ist und dort im Zusammenwirken mit dem oder den Haken (111, 112, 113, 114) eine Manipulation der zu manipulierenden Gewebeteile erlaubt.

2. Vorrichtung nach Anspruch 1,
**dadurch gekennzeichnet, dass** das Gleitelement (41) eine Beleuchtungseinrichtung aufweist, deren Lichtaustritt im Umfangsrand (43) der glockenförmigen Erweiterung (41) vorgesehen ist.

3. Vorrichtung nach einem der Ansprüche 1 bis 2,
**dadurch gekennzeichnet, dass** die glockenförmige Erweiterung (41) in etwa die Form einer Halbkugel hat.

4. Vorrichtung nach einem der Ansprüche 1 bis 3,
**dadurch gekennzeichnet, dass** eine Antriebseinheit für das Gleitelement (4) vorgesehen ist, die ein Handrad (5) aufweist, das eine in einer Längsnut geführte Kugel (51) über eine gewindeförmige Nut bewegt.

5. Vorrichtung nach einem der Ansprüche 1 bis 4,
**dadurch gekennzeichnet, dass** das Hakenelement (11) wenigstens drei, bevorzugt vier Haken (111, 112, 113, 114) aufweist, die bei geöffneten Griffstücken (31, 32) eingeklappt sind, und die durch das Schließen der Griffstücke (31, 32) durch die als Zugstange wirkende Stange (12) in Richtung auf das distale Ende ausgeklappt werden.

6. Vorrichtung nach einem der Ansprüche 1 bis 5,
**dadurch gekennzeichnet, dass** die Stange (12), mittels derer die Haken (111, 112, 113, 114) betätigt werden, mit den Griffstücken (31, 32) lösbar verbunden ist.

7. Vorrichtung nach einem der Ansprüche 4 bis 6,
**dadurch gekennzeichnet, dass** das Hakenelement (11), der Schaft (2), der Griff (3) und gegebenenfalls die Antriebseinheit voneinander trennbar sind.

## Claims

1. Device for carrying out manipulations in the human body, in particular in the uterus, comprising a hook element (11) including at least one hook connected to a handle (3), which is disposed on the proximal end, via a rod (12) guided in a shaft (2), so that it is adapted for being swing out and in, and comprising a slide element (41) guided on said shaft (2) for displacement in the longitudinal direction of the latter and presenting an approximately bell-shaped extended section (41) on its distal end,
**characterised in that** said bell-shaped extended section is configured on the distal end of said slide element (41) in such a way that it is adapted for being introduced into the human body where it cooperates with said hook or hooks (11₁, 11₂, 11₃, 11₄) for permitting a manipulation of the tissue segments to be manipulated.

2. Device according to Claim 1,
**characterised in that** said slide element (41) comprises an illuminating device whose light exit is provided in the peripheral edge (43) of said bell-shaped extended section (41).

3. Device according to any of the Claims 1 to 2,
**characterised in that** said bell-shaped extended section (41) has roughly the shape of a hemisphere.

4. Device according to any of the Claims 1 to 3,
**characterised in that** a drive unit is provided for said slide element (4), which includes a hand wheel moving a sphere (51) via a thread-like groove, which sphere is guided in a longitudinal groove.

5. Device according to any of the Claims 1 to 4,
**characterised in that** said hook element (11) includes at least three, preferably four hooks (11₁, 11₂, 11₃, 11₄) swung in when said grips (31, 32) are opened, which are swung out in a direction towards the distal end when said grips (31, 32) are closed by said rod (12) acting as a tie rod.

6. Device according to any of the Claims 1 to 5,
**characterised in that** said rod (12), by means of which said hooks (11₁, 11₂, 11₃, 11₄) are operated, is detachably connected to said grips (31, 32).

7. Device according to any of the Claims 4 to 6,
**characterised in that** said hook element (11), said shaft (2), said handle (3) and possibly said driving unit are adapted to be separated from each other.

## Revendications

1. Dispositif à effectuer des manipulations à l'intérieur du corps humain, notamment de l'utérus, comprenant un élément de griffe (11) à au moins une griffe reliée à une poignée (3), qui est disposée à l'extrémité proximale, via une barre (12) guidée dans une tige (2), de façon, qu'elle se prête à être basculée et escamotée, et comprenant un élément coulissant (41) guidée sur ladite tige (2) pour déplacement le long de la direction longitudinale de la dernière et comprenant une partie élargie environ sous forme de cloche (41) sur soin extrémité distale,
**caractérisé en ce que** ladite partie élargie sous forme de cloche est configurée sur l'extrémité distale dudit élément coulissant (41) de façon, qu'elle soit apte à être introduite dans le corps humain, où elle coopère avec ladite griffe ou lesdites griffes (11₁, 11₂, 11₃, 11₄) afin de permettre une manipulation des fragments de tissu à manipuler.

2. Dispositif selon la revendication 1,
**caractérisé en ce que** ledit élément coulissant (41) comprend un moyen d'éclairage, dont la sortie de lumière est formée dans le bord périphérique (43) de ladite partie élargie sous forme de cloche (41).

3. Dispositif selon une quelconque des revendications 1 à 2,
**caractérisé en ce que** ladite partie élargie sous forme de cloche (41) présente environ la forme d'un hémisphère.

4. Dispositif selon une quelconque des revendications 1 à 3,
**caractérisé en ce qu'**une unité d'entraînement est prévue pour ledit élément coulissant (4), qui comprend un volant à mouvoir une sphère (51) via une rainure sous forme de filet, cette sphère étant guidée dans une rainure longitudinale.

5. Dispositif selon une quelconque des revendications 1 à 4,
**caractérisé en ce que** ledit élément de griffe (11) comprend au moins trois, de préférence quatre, griffes (11₁, 11₂, 11₃, 11₄), qui sont escamotées, quand lesdites anses (31, 32) sont ouvertes, et qui sont basculées en une direction vers l'extrémité distale, quand lesdites anses (31, 32) sont fermées par ladite barre (12), qui agit en tant que tirant.

6. Dispositif selon une quelconque des revendications 1 à 5,
**caractérisé en ce que** ladite barre (12), moyennant laquelle lesdites griffes (11₁, 11₂, 11₃, 11₄) sont commandées, est reliée auxdites anses (31, 32) de façon amovible.

7. Dispositif selon une quelconque des revendications 4 à 6,
**caractérisé en ce que** ledit élément de griffe (11), ladite tige (2), ladite poignée (3) et éventuellement ladite unité d'entraînement sont aptes à être séparés l'un de l'autre.
